# EUROPÄISCHE PATENTSCHRIFT

(11) **EP 4 541 313 B1**
(45) Veröffentlichungstag und Bekanntmachung des Hinweises auf die Patenterteilung: **12.08.2026**
(21) Anmeldenummer: 23204474.3
(22) Anmeldetag: 18.10.2023
(51) Int. Cl.: A61F 2/18

(54) **GEHÖRKNÖCHELCHENPROTHESE**
AUDITORY OSSICLE PROSTHESIS
PROTHÈSE D'OSSELETS DE L'OREILLE

(43) Veröffentlichungstag der Anmeldung: 23.04.2025
(73) Patentinhaber: MED-EL Elektromedizinische Geräte GmbH, 6020 Innsbruck (AT)
(72) Erfinder: Kuen, Clemens, 6460 Imst (AT); Steinhardt, Uwe, 72145 Hirrlingen (DE); Riedl, Lukas, 6130 Schwaz (AT); Casagrande, Giade, 6020 Innsbruck (AT)
(74) Vertreter: Witte, Weller & Partner Patentanwälte mbB

(56) Entgegenhaltungen:
- DE-B3- 102010 046 457
- DE-U1- 20 212 771
- US-A1- 2008 097 603

## Beschreibung

Die Erfindung betrifft eine Gehörknöchelchenprothese mit den Merkmalen des Oberbegriffs des Anspruchs 1.

Gehörknöchelchenprothesen dienen als Ersatz eines oder mehrerer Gehörknöchelchen im menschlichen Mittelohr und übertragen anstatt des oder der ersetzten Gehörknöchelchen durch Schallwellen erzeugte Schwingungen des Trommelfells auf ein vorhandenes Gehörknöchelchen, meist auf den Steigbügel. Möglich ist auch eine Übertragung einer Schwingung eines künstlichen, in ein menschliches Ohr implantierten Schwingungserzeugers auf das Gehörknöchelchen.

Das Gebrauchsmuster DE 202 12 771 U1 offenbart eine Gehörknöchelchenprothese, die eine aus einem Federmetallstreifen gebogene, elastisch aufweitbare Federklemme zu einer Befestigung an einem Gehörknöchelchen aufweist. Die Federklemme weist zwei einander gegenüberliegende, bogenförmig nach außen gewölbte Klemmabschnitte und eine Öffnung zum Aufsetzen der Federklemme auf das Gehörknöchelchen auf einer Seite der Klemmabschnitte auf. Ist die Federklemme wie vorgesehen auf das Gehörknöchelchen aufgesetzt, liegen die Klemmabschnitte der Federklemme an gegenüberliegenden Umfangsstellen des Gehörknöchelchens an. Wird die Federklemme der bekannten Gehörknöchelchenprothese beim Aufsetzen auf das Gehörknöchelchen elastisch aufgeweitet, entfernen sich die beiden bogenförmigen Klemmabschnitte nicht nur voneinander, sondern sie schwenken auch derart auseinander, dass die Klemmabschnitte ihren einschließenden Winkel zueinander ändern. Auf einem Gehörknöchelchen mit größerem Durchmesser stehen die Klemmabschnitte der Federklemme daher V-förmig schräg zueinander. Die Federrückstellkraft bewirkt damit eine Kraftkomponente in Richtung der Öffnung der Gehörknöchelchenprothese, sodass die Stabilität der Klemmfixierung beeinträchtigt wird.

Das Patent DE 10 2010 046 457 B3 offenbart eine Gehörknöchelchenprothese mit einer U-förmigen Federklemme zum Aufsetzen auf ein Endstück eines Schwingungen erzeugenden Aktors. Die Federklemme weist zueinander parallele Schenkel mit einander gegenüberliegenden, nach außen gewölbten Wellungen auf. Durch Aufschnappen der Federklemme mit verschiedenen, gegenüberliegenden Wellungen auf das Endstück des Aktors lässt sich die Federklemme in ihrer Längsrichtung auf dem Endstück des Aktors verstellen und auf diese Weise die Gehörknöchelchenprothese an einen Abstand des Endstücks des Aktors von einem Gehörknöchelchen anpassen. Auch diese Federklemme federt bei einem Aufweiten V-förmig auseinander derart, dass die Schenkel bei aufgefederten Federklemme schräg zueinander stehen.

Aufgabe der Erfindung ist, eine Gehörknöchelchenprothese mit einer elastisch aufweitbaren Federklemme zum Aufsetzen auf ein Gehörknöchelchen vorzuschlagen, deren Sitz auf Gehörknöchelchen mit unterschiedlichen Durchmessern verbessert ist.

Diese Aufgabe wird erfindungsgemäß durch die Merkmale des Anspruchs 1 gelöst. Die erfindungsgemäße Gehörknöchelchenprothese mit den Merkmalen des Anspruchs 1 weist eine elastisch aufweitbare Federklemme zu einer Befestigung der Gehörknöchelchenprothese an einem Gehörknöchelchen auf. Die Federklemme weist zwei einander gegenüber angeordnete Klemmbacken zu einem Festklemmen der Federklemme auf dem Gehörknöchelchen und eine Öffnung zu einem Aufsetzen der Federklemme auf das Gehörknöchelchen auf einer Seite der Klemmbacken auf. Ist die Federklemme wie vorgesehen auf das Gehörknöchelchen aufgesetzt, liegen die beiden Klemmbacken an im wesentlichen gegenüberliegenden Umfangsstellen des Gehörknöchelchens an.

Auf einer der Öffnung gegenüberliegenden Seite der Klemmbacken weist die Federklemme der erfindungsgemäßen Gehörknöchelchenprothese einen radialelastischen Federbügel auf, an dem, insbesondere an seinen Enden die Klemmbacken angeordnet sind. Beispielsweise erstreckt sich der Federbügel bogenförmig mit einer Krümmung nach außen von einer zu einer anderen der beiden Klemmbacken der Federklemme. "Radialelastisch" bedeutet, dass der Federbügel radial zu dem Gehörknöchelchen federt, auf das die Federklemme aufgesetzt ist. Der Federbügel beaufschlagt die beiden Klemmbacken elastisch von außen gegen die gegenüberliegenden Umfangsstellen des Gehörknöchelchens, wenn die Federklemme wie vorgesehen auf das Gehörknöchelchen aufgesetzt ist.

Erfindungsgemäß weist der Federbügel eine sich in einer Umfangsrichtung beziehungsweise von der einen zu der anderen Klemmbacke vergrößernde Krümmung auf. Durch eine Form der Krümmung des Federbügels lässt sich erreichen, dass sich bei dem elastischen Aufweiten der Federklemme die beiden Klemmbacken voneinander entfernen ohne ihren einschließenden Winkel zueinander zu verändern oder mit einer verhältnismäßig kleinen Änderung ihres Winkels zueinander. Das bedeutet, dass zwei zueinander parallele Klemmbacken bei dem elastischen Aufweiten der Federklemme parallel zueinander bleiben oder nur in einen kleinen Winkel zueinander schwenken. Ein Schwenkwinkel der beiden Klemmbacken der Federklemme beim elastischen Aufweiten der Federklemme ist kleiner als es bei einem kreisbogenförmigen Federbügel mit konstanter Krümmung oder auch bei einem V- oder U-förmigen Federbügel der Fall wäre. Ein Sitz der Federklemme der erfindungsgemäßen Gehörknöchelchenprothese auf Gehörknöchelchen mit unterschiedlichen Durchmessern, die die Federklemme unterschiedlich weit Aufweiten, ist dadurch verbessert. In der Umfangsrichtung erstreckt sich der Federbügel beispielsweise über etwa 180°.

Die abhängigen Ansprüche haben vorteilhafte Ausgestaltungen und Weiterbildungen der Erfindung zum Gegenstand.

Vorzugsweise weist der Federbügel der Federklemme der erfindungsgemäßen Gehörknöchelchenprothese die Form einer hyperbolischen Spirale oder einer logarithmischen Spirale oder eine ähnliche Form auf. Diese Form der Federklemme ermöglicht ein Entfernen der beiden Klemmbacken bei dem elastischen Aufweiten der Federklemme ohne Verschwenken der Klemmbacken gegeneinander oder mit einem kleinen Schwenkwinkel.

Eine Ausgestaltung der Erfindung sieht vor, dass einander diametral gegenüberliegende Enden der beiden Klemmbacken an Enden des Federbügels angeordnet sind. Es ist also ein der Öffnung der Federklemme nahes Ende einer der beiden Klemmbacken, das öffnungsseitige Ende, an einem Ende des Federbügels und ein der Öffnung der Federklemme fernes Ende der anderen Klemmbacke an einem anderen Ende des Federbügels angeordnet. Beispielsweise umgreift der Federbügel eine der beiden Klemmbacken auf ihrer der anderen Klemmbacke abgewandten Außenseite und erstreckt sich außen um ein der Öffnung der Federklemme fernes Ende dieser Klemmbacke herum zu der anderen Klemmbacke. Diese Anordnung der Klemmbacken am Federbügel bewirkt ebenfalls ein in etwa paralleles Entfernen der beiden Klemmbacken voneinander bei dem elastischen Aufweiten der Federklemme beziehungsweise einen kleinen Schwenkwinkel der beiden Klemmbacken, wenn sich die beiden Klemmbacken bei dem elastischen Aufweiten der Federklemme voneinander entfernen. Diese Ausgestaltung der Erfindung ist auch bei Ausführungen der Federklemme möglich, deren Federbügel eine andere Form als im Anspruch 1 angegebene Form oder nicht die Form einer hyperbolischen Spirale, einer logarithmischen Spirale oder keine Spiralform aufweist.

Eine Ausgestaltung der Erfindung sieht einen mit den Klemmbacken einstückigen Federbügel vor. Bei dieser Ausführung der Erfindung sind die Klemmbacken beispielsweise Umfangsabschnitte beziehungsweise Längsabschnitte des Federbügels. Beispielsweise durch eine größere Dicke können die Klemmbacken bildende Abschnitte des Federbügels starr oder jedenfalls steifer als ein die Klemmbacken verbindender Abschnitt des Federbügels sein.

In einer weiteren Ausgestaltung der Erfindung weist die Gehörknöchelchenprothese einen Schaft und eine Federklemme an einem Ende des Schafts auf. Der Schaft dient zu einer Übertragung mechanischer Schwingungen von der Federklemme an das andere Ende des Schafts, welches ausgebildet ist, um zumindest teilweise beispielsweise am ovalen Fenster in das Innenohr einführbar zu sein. Damit werden mechanische Schwingungen an die Flüssigkeit des Innenohrs abgegeben. Eine Gehörknöchelchenprothese mit dieser Ausgestaltung wird auch Steigbügelprothese genannt, weil sie den Steigbügel der drei Gehörknöchelchen ersetzt.

Eine Ausgestaltung der Erfindung sieht einen Abstand der öffnungsseitigen Enden der Klemmbacken bei entspanntem Federbügel von zumindest 0,5mm vor. In einer weiteren Ausgestaltung der Erfindung weist die Federklemme einen Fortsatz auf. Der Fortsatz weist bevorzugt eine Länge von zumindest 0,6mm auf.

Sämtliche in der Beschreibung genannten und/oder der Zeichnung dargestellten Merkmale können einzeln für sich oder in jeder beliebigen Kombination bei Ausführungen der Erfindung verwirklicht sein. Ausführungen der Erfindung, die nicht alle, sondern nur einen Teil der Merkmale eines Anspruchs, auch des unabhängigen Anspruchs, aufweisen, sind möglich.

Die Erfindung wird nachfolgend anhand eines in der Zeichnung dargestellten Ausführungsbeispiels näher erläutert. Es zeigen:
Figur 1 zeigt eine Gehörknöchelchenprothese gemäß einer Ausführungsform der Erfindung.
Figur 2 A-C zeigt die Federklemme gemäß einer Ausführungsform der Erfindung in drei unterschiedlich stark elastisch aufgeweiteten Positionen.

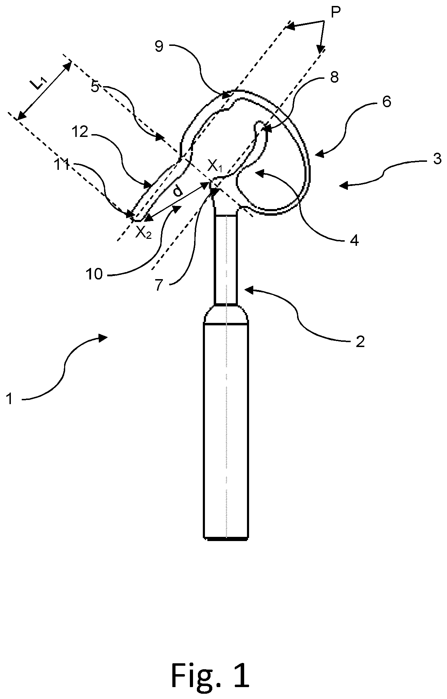

Die in Figur 1 dargestellte, erfindungsgemäße Gehörknöchelchenprothese 1 ist zu einem Ersatz eines oder mehrerer Gehörknöchelchen in einem menschlichen Mittelohr vorgesehen. Die Gehörknöchelchenprothese 1 weist einen Schaft 2 und eine Federklemme 3 an einem Ende des Schafts 2 auf. Der Schaft 2 dient zu einer Übertragung mechanischer Schwingungen von einem Gehörknöchelchen auf das Innenohr oder auch zu einer Übertragung mechanischer Schwingungen eines in ein menschliches Ohr implantierten Schwingungserzeugers oder Schallwandlers auf ein Gehörknöchelchen. Der Schaft 2 wird in diesem Fall an dem Schwingungserzeuger oder Schallwandler angebracht. Die Federklemme 3 wird auf das Gehörknöchelchen aufgesetzt. Der Schaft 2 kann auch an jedem Ende eine Federklemme 3 aufweisen (nicht dargestellt).

Die Federklemme 3 weist zwei einander in einem Abstand gegenüber angeordnete, biegesteife Klemmbacken 4, 5 auf, die bezüglich eines Zwischenraums zwischen den Klemmbacken 4, 5 konkav und ungefähr entsprechend einem Durchmesser des Gehörknöchelchens oder des Teils des Gehörknöchelchens, auf das die Federklemme 3 aufgesetzt werden soll, gewölbt sind. Es sind jedenfalls einander zugewandte Innenseiten der Klemmbacken 4, 5 konkav bezüglich des Zwischenraums gewölbt. Weil Gehörknöchelchen individuell sind, kann die Wölbung der Klemmbacken 4, 5 nicht genau festgelegt werden, es sei denn, sie werden individuell angepasst.

Außer den beiden Klemmbacken 4, 5 weist die Federklemme 3 einen radialelastischen Federbügel 6 auf, der die beiden Klemmbacken 4, 5 elastisch derart verbindet, dass der Abstand der beiden Klemmbacken 4, 5 voneinander elastisch veränderbar ist. "Radialelastisch" bedeutet, dass der Federbügel 6 vergleichbar einer gewölbten Blattfeder in einer Radialebene zu einer gedachten Achse durch eine Mitte oder durch ein Zentrum zwischen den beiden Klemmbacken 4, 5 elastisch verformbar ist.

Ein Ende des Federbügels 6 ist mit einem Ende 7 einer der beiden Klemmbacken 4 verbunden und führt bogenförmig um eine der anderen Klemmbacke 5 abgewandte Außenseite der einen Klemmbacke 4 und um ein anderes Ende 8 der einen Klemmbacke 4 herum zu einem Ende 9 der anderen Klemmbacke 5, das mit dem anderen Ende des Federbügels 6 verbunden ist. Es sind in diesem Beispiel damit einander diametral gegenüberliegende Enden 7, 9 oder 8, 11 der beiden Klemmbacken 4, 5 mit den Enden des Federbügels 6 verbunden.

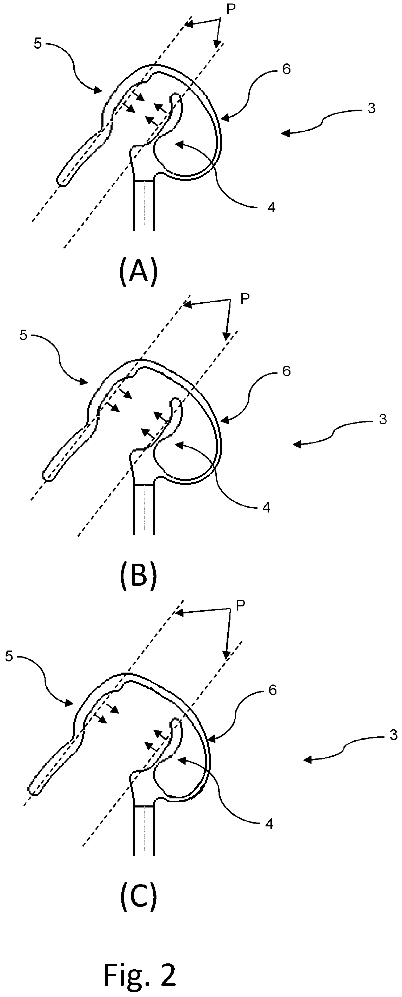

Der Federbügel 6 der Federklemme 3 der erfindungsgemäßen Gehörknöchelchenprothese 1 in diesem Beispiel erstreckt sich über einen Umfangswinkel von etwa 180° von dem einen Ende 7 der einen Klemmbacke 4 zu dem diametral gegenüberliegenden Ende 9 der anderen Klemmbacke 5. Ein Krümmungsradius des Federbügels 6 vergrößert oder verkleinert sich - insbesondere stetig - von dem einen Ende 7 der einen Klemmbacke 4 zu dem diametral gegenüberliegenden Ende 9 der anderen Klemmbacke 5. Diese Formgebung des Federbügels 6 bewirkt, dass sich beim elastischen Aufweiten der Federklemme 3 der Drehpunkt stetig derart verändert, sodass sich die Klemmbacken 4 und 5 relativ zueinander parallel oder nahezu parallel bewegen. Die parallele oder nahezu parallele Anordnung der Klemmbacken 4 und 5 ist in Figur 1 sowie Figuren 2 (A) bis (C) mit den Strichlinien P dargestellt. Im Ausführungsbeispiel ist der Krümmungsradius des Federbügels 6 auf der der anderen Klemmbacke 5 gegenüberliegenden Außenseite der einen Klemmbacke 4 nur etwa halb so groß wie eine Erstreckung der einen Klemmbacke 4 in der Umfangsrichtung. Anders ausgedrückt ist der Federbügel 6 ausgehend von dem einen Ende 7 der einen Klemmbacke 4 auf einem Umfangsabschnitt von etwas weniger als 180° näherungsweise kreisförmig. Im restlichen Bereich bis in diesem Beispiel zu dem diametral gegenüberliegenden Ende 9 der anderen Klemmbacke 5 weist der Federbügel 6 die Form einer Blattfeder mit kleiner werdender Krümmung auf. Beispielsweise weist der Federbügel 6 die Form einer hyperbolischen Spirale oder einer logarithmischen Spirale oder eine ähnliche Form auf.

In einem Bereich, der um das andere Ende 8 der einen Klemmbacke 4 herum zu dem einen Ende 9 der anderen Klemmbacke 5 führt, erhöht sich im dargestellten Ausführungsbeispiel der Erfindung eine Biegesteifigkeit des Federbügels 6 - im Ausführungsbeispiel durch eine in Richtung der anderen Klemmbacke 5 zunehmende radiale Dicke des Federbügels 6.

Durch die Spiralform oder die einer Spirale ähnliche Form des Federbügels 6 bewegen sich die Klemmbacken 4, 5 parallel oder näherungsweise parallel zueinander, wenn die Federklemme 3 elastisch aufgeweitet wird, das heißt der Abstand der beiden Klemmbacken 4, 5 voneinander vergrößert wird. Das heißt bei dem elastischen Aufweiten der Federklemme 3 schwenken die beiden Klemmbacken 4, 5 nicht oder nur wenig, sondern bewegen sich im Wesentlichen relativ parallel auseinander. Das elastische Aufweiten der Federklemme 3 durch die radialelastische Aufbiegung des Federbügels 6, bei dem sich die beiden Klemmbacken 4, 5 parallel oder nahezu parallel voneinander entfernen, ist in den Figuren 2 (A) bis (C) mit Pfeilen in etwa rechtwinkelig zu den Strichlinien P dargestellt. Die Gehörknöchelchenprothese 1 kann kompakter gebaut werden, indem die beiden Enden des Federbügels 6 zu einander diametral gegenüberliegenden Enden 7, 9 oder 8, 11 der beiden Klemmbacken 4, 5 angeordnet werden. Kompakt bedeutet dabei, dass der Federbügel 6 nicht zu weit von den Klemmbacken 4, 5 absteht und die Federklemme 3 insgesamt vergrößert. Das hat den Vorteil, dass die Gehörknöchelchenprothese einfacher im Mittelohrraum platziert werden kann.

Die Federklemme 3 ist einstückig, das heißt die beiden Klemmbacken 4, 5 sind einstückig mit dem Federbügel 6. Die Klemmbacken 4, 5 und der Federbügel 6 befinden sich in einer Ebene, die die Radialebene ist, in der die Federklemme 3 radialelastische aufweitbar ist und die im Ausführungsbeispiel eine Axialebene des Schafts 2 ist.

Im Ausführungsbeispiel ist die Federklemme 3 an oder nahe an dem einen Ende 7 der einen Klemmbacke 4, an dem das eine Ende des Federbügels 6 angeordnet ist, an dem einen Ende des Schafts 2 angeordnet, der in etwa radial nach außen von der Federklemme 3 absteht.

Auf einer dem Federbügel 6 gegenüberliegenden Seite weist die Federklemme 3 eine Öffnung 10 zwischen den Enden 7 der beiden Klemmbacken 4, 5 auf. Durch die Öffnung 10 lässt sich die Federklemme 3 auf das nicht dargestellte Gehörknöchelchen aufsetzen, wobei sich die Federklemme 3 elastisch aufweitet und die einander zugewandten Innenseiten ihrer Klemmbacken 4, 5 elastisch von außen gegen das Gehörknöchelchen drückt derart, dass die Federklemme 3 auf dem Gehörknöchelchen festgeklemmt ist.

Das eine Ende des Federbügels 6 ist an dem einen, der Öffnung 10 des Federbügels 6 nahen Ende 7, dem öffnungsseitigen Ende 7, der einen Klemmbacke 4 angeordnet und das andere Ende des Federbügels 6 ist an dem diametral gegenüberliegenden, nämlich der Öffnung 10 des Federbügels 6 fernen Ende 9 der anderen Klemmbacke 5 angeordnet.

An dem Ende der anderen Klemmbacke 5, das dem Federbügel 6 abgewandt ist, dem öffnungsseitigen Ende, weist die Federklemme 3 einen Fortsatz 12 in Verlängerung der anderen Klemmbacke 5 auf, der das Aufsetzen der Federklemme 3 auf das Gehörknöchelchen erleichtert. In einer vorteilhaften Ausführungsform weist der Fortsatz 12 eine Länge von zumindest 0,6mm auf, gemessen von dem Punkt auf der Klemmbacke 5, der den geringsten öffnungsseitigen Abstand zur Klemmbacken 4 aufweist (nicht eingezeichnet in den Figuren).

In einem Ausführungsbeispiel ist der innenseitige Abstand der öffnungsseitigen Enden der Klemmbacken 4 und 5 bei entspanntem Federbügel 6 zumindest 0,5mm. In der Figur 1 ist dieser Abstand zwischen den Punkten X₁ und X₂ mit d markiert. Ist sowohl der Fortsatz 12 zumindest 0.6mm lang und der innenseitige Abstand der öffnungsseitigen Enden der Klemmbacken 4 und 5 bei entspanntem Federbügel 6 zumindest 0,5mm, so hat das den Vorteil, dass das Aufsetzen auf ein Gehörknöchelchen der elastisch aufweitbaren Federklemme 3 wesentliche vereinfacht wird. In der in Figur 1 gezeigten Ausführungsform etwa wird das Gehörknöchelchen zwischen Schaft 2 und Fortsatz 12 geradezu durch die Öffnung geführt.

## Patentansprüche

1. Gehörknöchelchenprothese mit einer zum Aufsetzen auf ein Gehörknöchelchen elastisch aufweitbaren Federklemme (3), die Federklemme (3) umfassend zwei einander gegenüberliegend angeordnete Klemmbacken (4, 5) zum Festklemmen der Federklemme (3) auf dem Gehörknöchelchen; eine Öffnung (10) zum Aufsetzen der Federklemme (3) auf das Gehörknöchelchen sodass die beiden Klemmbacken (4, 5) an im wesentlichen gegenüberliegenden Umfangsstellen des Gehörknöchelchens anliegen können, und; einen radialelastischen Federbügel (6) der die Klemmbacken (4, 5) auf einer der Öffnung (10) gegenüberliegenden Seite elastisch zueinander beweglich verbindet, **dadurch gekennzeichnet, dass** der Federbügel (6) eine sich in einer Umfangsrichtung von einer zu der jeweiligen anderen der beiden Klemmbacken (4, 5) verändernde Krümmung derart aufweist, sodass bei einem elastischen Aufweiten der Federklemme (3), beispielsweise beim Aufsetzen auf das Gehörknöchelchen, sich die Klemmbacken (4, 5) relativ zueinander parallel oder nahezu parallel bewegen.

2. Gehörknöchelchenprothese nach Anspruch 1, **dadurch gekennzeichnet, dass** der Federbügel (6) eine kontinuierlich verlaufend vergrößernde oder verkleinernde Krümmung aufweist.

3. Gehörknöchelchenprothese nach Anspruch 1 oder 2, **dadurch gekennzeichnet, dass** der Federbügel (6) die Form einer hyperbolischen Spirale oder einer logarithmischen Spirale aufweist.

4. Gehörknöchelchenprothese nach einem oder mehreren der Ansprüche 1 bis 3, **dadurch gekennzeichnet, dass** einander diametral gegenüberliegende Enden (7, 9 oder 8, 11) der beiden Klemmbacken (4, 5) an Enden des Federbügels (6) angeordnet sind.

5. Gehörknöchelchenprothese nach einem oder mehreren der Ansprüche 1 bis 4, **dadurch gekennzeichnet, dass** die Klemmbacken (4, 5) einstückig mit dem Federbügel (6) sind.

6. Gehörknöchelchenprothese nach einem oder mehreren der Ansprüche 1 bis 5, **dadurch gekennzeichnet, dass** die Gehörknöchelchenprothese einen Schaft (2) aufweist, wobei die Federklemme (3) an einem Ende des Schafts (2) angeordnet ist und der Schaft (2) an dem anderen Ende ausgebildet um zumindest teilweise in das Innenohr einführbar ist, sodass mechanischer Schwingungen von der Federklemme (3) an das Innenohr übertragen werden können.

7. Gehörknöchelchenprothese nach einem oder mehreren der vorstehenden Ansprüche, **dadurch gekennzeichnet, dass** der Abstand der öffnungsseitigen Enden der Klemmbacken (4, 5) bei entspanntem Federbügel (6) zumindest 0,5mm beträgt.

8. Gehörknöchelchenprothese nach einem oder mehreren der vorstehenden Ansprüche, **dadurch gekennzeichnet, dass** die Federklemme (3) einen Fortsatz (12) aufweist.

9. Gehörknöchelchenprothese nach Anspruch 8, **dadurch gekennzeichnet, dass** der Fortsatz (12) eine Länge von zumindest 0,6mm aufweist.

## Claims

1. Ossicular prosthesis with a spring clamp (3) that can be elastically expanded for placement on an ossicle, the spring clamp (3) comprising two opposing clamping jaws (4, 5) for clamping the spring clamp (3) onto the ossicle, an opening (10) for placing the spring clamp (3) onto the ossicle so that the two clamping jaws (4, 5) can rest against substantially opposite circumferential points of the ossicle, and a radially elastic spring clip (6) which elastically connects the clamping jaws (4, 5) to each other on a side opposite of the opening (10), **characterized in that** the spring clip (6) has a curvature that varies in a circumferential direction from one to the respective other of the two clamping jaws (4, 5) such that upon elastic expansion of the spring clamp (3), for example when placed on the ossicle, the clamping jaws (4, 5) move parallel or almost parallel relative to each other.

2. Ossicular prosthesis according to claim 1, **characterized in that** the spring clip (6) has a continuously increasing or decreasing curvature.

3. Ossicular prosthesis according to claim 1 or 2, **characterized in that** the spring clip (6) has the shape of a hyperbolic spiral or a logarithmic spiral.

4. Ossicular prosthesis according to one or more of claims 1 to 3, **characterized in that** diametrically opposite ends (7, 9 or 8, 11) of the two clamping jaws (4, 5) are arranged at ends of the spring clip (6).

5. Ossicular prosthesis according to one or more of claims 1 to 4, **characterized in that** the clamping jaws (4, 5) are formed in one piece with the spring clip (6).

6. Ossicular prosthesis according to one or more of claims 1 to 5, **characterized in that** the ossicular prosthesis comprises a shaft (2), wherein the spring clamp (3) is arranged at one end of the shaft (2) and the shaft (2) is formed at the other end to be at least partially insertable into the inner ear, so that mechanical vibrations can be transmitted from the spring clamp (3) to the inner ear.

7. Ossicular prosthesis according to one or more of the preceding claims, **characterized in that** the distance between the opening-side ends of the clamping jaws (4, 5) is at least 0.5 mm when the spring clip (6) is relaxed.

8. Ossicular prosthesis according to one or more of the preceding claims, **characterized in that** the spring clamp (3) comprises a prolongation (12).

9. Ossicular prosthesis according to claim 8, **characterized in that** the prolongation (12) has a length of at least 0.6 mm.

## Revendications

1. Prothèse des osselets de l'oreille comportant une pince à ressort (3) dilatable élastiquement pour être placée sur un osselet de l'oreille, la pince à ressort (3) comprenant deux mâchoires de serrage (4, 5) disposées en vis-à-vis pour bloquer la pince à ressort (3) sur l'osselet de l'oreille, une ouverture (10) pour la mise en place de la pince à ressort (3) sur l'osselet de l'oreille de manière à ce que les deux mâchoires de serrage (4, 5) puissent s'appuyer contre des points circonférentiels de l'osselet de l'oreille essentiellement opposés, et un étrier à ressort (6) élastique dans le sens radial qui relie les mâchoires de serrage (4, 5) à un lieu opposé de l'ouverture (10) de manière à ce qu'elles puissent se déplacer élastiquement l'une par rapport à l'autre, **caractérisée en ce que** l'étrier à ressort (6) a une courbure variant dans une direction circonférentielle de l'une à l'autre des deux mâchoires de serrage (4, 5) de sorte que lorsque la pince à ressort (3) se dilate élastiquement, par exemple lorsqu'elle est placée sur l'osselet de l'oreille, les mâchoires de serrage (4, 5) se déplacent parallèlement ou presque parallèlement l'une à l'autre.

2. Prothèse des osselets de l'oreille selon la revendication 1, **caractérisée en ce que** l'étrier à ressort (6) a une courbure continuellement croissante ou décroissante.

3. Prothèse des osselets de l'oreille selon la revendication 1 ou 2, **caractérisée en ce que** l'étrier à ressort (6) a la forme d'une spirale hyperbolique ou d'une spirale logarithmique.

4. Prothèse des osselets de l'oreille selon une ou plusieurs des revendications 1 à 3, **caractérisée en ce que** les extrémités diamétralement opposées (7, 9 ou 8, 11) des deux mâchoires de serrage (4, 5) sont disposées aux extrémités de l'étrier à ressort (6).

5. Prothèse des osselets de l'oreille selon une ou plusieurs des revendications 1 à 4, **caractérisée en ce que** les mâchoires de serrage (4, 5) sont réalisées d'une seule pièce avec l'étrier à ressort (6).

6. Prothèse des osselets de l'oreille selon une ou plusieurs des revendications 1 à 5, **caractérisée en ce que** la prothèse ossiculaire comporte une tige (2), la pince à ressort (3) est disposée à une extrémité de la tige (2) et l'autre extrémité de la tige (2) est conçue pour être au moins partiellement insérable dans l'oreille interne, de manière que vibrations mécaniques peuvent être transmises de la pince à ressort (3) à l'oreille interne.

7. Prothèse des osselets de l'oreille selon une ou plusieurs des revendications précédentes, **caractérisée en ce que** la distance entre les extrémités des mâchoires de serrage (4, 5) à côté d'ouverture (10) est d'au moins 0,5 mm lorsque l'étrier à ressort (6) est relâché.

8. Prothèse des osselets de l'oreille selon une ou plusieurs des revendications précédentes, **caractérisée en ce que** la pince à ressort (3) a un prolongement (12).

9. Prothèse des osselets de l'oreille selon la revendication 8, **caractérisée en ce que** le prolongement (12) a une longueur d'au moins 0,6 mm.
